# EUROPEAN PATENT APPLICATION

(11) **EP 1 818 043 A2**
(43) Date of publication of application: **15.08.2007**
(21) Application number: 07001588.8
(22) Date of filing: 25.01.2007
(51) Int. Cl.: A61K 8/73, A61K 8/81, A61Q 3/02

(54) **Nail compositions in gelled form**

(30) Priority: 13.02.2006 US 352643
(71) Applicant: L'Oréal, 75008 Paris (FR)
(72) Inventor: Weber, Robert, Suffern, NY 10901 (US); Patel, Nishith, Rosellen Park, NJ 07204 (US)
(74) Representative: Le Coupanec, Pascale A.M.P.

(57) **Abstract**

A process for making a nail polish varnish involving the steps of:(a) providing a gelled nail polish composition having a Brookfield viscosity of at least 20 poise, the composition containing: (i) at least one gelling agent; (ii) at least one film forming polymer; (iii) at least one solvent; and (iv) at least one colorant; (b) providing at least one chemical viscosity reducing agent; and (c) combining the gelled nail polish composition, and the at least one chemical viscosity reducing agent, to form a nail polish varnish having a Brookfield viscosity of at most 30 poise.

## Description

Nail polish varnishes are typically made available to consumers in a variety of pre-made colors, shades and styles. Because these products require the use of large amounts of volatiles, they need to be packaged in small glass containers which, by their very nature, are breakable, thus requiring special handling procedures. Thus, due to this packaging, retailers, professional salons and consumers need to exercise care in the handling and storage of nail polish varnishes.

A need, therefore, exists for a nail polish composition and system which requires little, if any, special handling/storage procedures.

Moreover, conventional nail polish varnishes require the application of at least two coats of varnish onto the nail in order to obtain satisfactory coverage on the nail. This, in turn, results in the expenditure of additional time and effort in making up nails.

Thus, it is also an object of the present invention to provide a system for making up nails which requires only one-coat coverage.

The present invention is directed to a process for making a nail polish varnish comprising at least the steps of: (a) providing a gelled nail polish composition having a Brookfield viscosity of at least 20 poise, the composition containing: (i) at least one gelling agent; (ii) at least one film-forming polymer; (iii) at least one solvent; and (iv) optionally, at least one colorant; (b) providing at least one chemical viscosity reducing agent; and (c) combining the gelled nail polish composition and the at least one chemical viscosity reducing agent to form a nail polish varnish having a Brookfield viscosity of at most 30 poise.

The present invention is also directed to a nail polish varnish composition containing: (a) at least one gelling agent; (b) at least one film-forming polymer; (c) at least one solvent; (d) optionally, at least one colorant; and (e) at least one chemical viscosity reducing agent, wherein (a) and (d) are present in the composition in an amount sufficient to facilitate one-coat coverage of the nail polish varnish on nails.

According to yet another embodiment, the present invention relates to a process for making up nails comprising at least : (a) providing a gelled nail polish composition having a Brookfield viscosity of at least 20 poise, the composition containing: (i) at least one gelling agent; (ii) at least one film-forming polymer; (iii) at least one solvent; (iv) and optionally, at least one colorant; (b) providing at least one chemical viscosity reducing agent; (c) combining the gelled nail polish composition and the at least one chemical viscosity reducing agent to form a nail polish varnish having a Brookfield viscosity of at most 30 poise; and (d) coating at least a nail with the nail polish varnish, wherein (c) may be performed just prior to step (d).

Another aspect of the present invention relates to a cosmetic kit for making up nails and/or to a multi-unit kit for making custom-colored nail polish varnish, the kit comprising: (a) at least one unit having a gelled nail polish composition having a viscosity of at least 20 poise, the composition containing: (i) at least one gelling agent; (ii) at least one film-forming polymer; (iii) at least one solvent; (iv) optionally, at least one colorant; (b) at least one unit containing at least one chemical viscosity reducing agent capable of reducing the viscosity of the gelled nail polish composition; (c) optionally, at least one container in which (a) and (b) can be blended; (d) optionally, at least tool for blending (a) and (b); and (e) optionally, at least one tool for coating nails.

Other than in the operating examples, or where otherwise indicated, all numbers expressing quantities of ingredients and/or reaction conditions are to be understood as being modified in all instances by the term "about".

All Brookfield viscosity values referenced herein are measured using a T-E or T-F spindle, at 6 rpm, at room temperature, for a period of 10 minutes.

Gelling Agent

The gelled nail polish composition according to the invention comprises a gelling agent in a sufficient quantity to carry a pre-determined concentration of colorant. The gelling agent must be capable of exhibiting a drop in viscosity in response to a change in its environment such as, for example, chemical, mechanical or thermal input. Changes in polarity and concentration may also be employed in order to effectuate the drop in viscosity.

The gelling agent may be hydrophilic for an aqueous medium, or lipophilic for an oily medium.

Examples of suitable gelling agents for aqueous mediums include, but are not limited to, hydrophilic clays, and hydrophilic fumed silica.

Clays are silicates comprising a cation which may be chosen from calcium, magnesium, aluminum, sodium, potassium and lithium cations, and mixtures thereof.

Suitable hydrophilic clays may include, but are not limited to, clays of the family of smectites such as montmorillonites, hectorites, bentonites, beidellites and saponites, and of the family of vermiculites, stevensite, chlorites, and synthetic hectorites (also called laponites), bentonites, magnesium and aluminum, and calcium silicates, and their mixtures.

Suitable hydrophilic fumed silicas may include, but are not limited to, those commercially available under the tradenames "AEROSIL^{®}", by the company Degussa, and "CAB-O-SIL^{®}" by the company Cabot.

Examples of suitable gelling agents for the oily medium may be chosen from organophilic clays, hydrophobic fumed silicas, modified cellulose derivatives, and elastomeric organopolysiloxanes.

The organophilic clays are clays modified by chemical compounds making the clay capable of swelling in oily media.

Suitable organophilic clays may include, but are not limited to, montmorillonite, bentonite, hectorite, attapulgite, sepiolite, and mixtures thereof.

These clays may be modified with a chemical compound chosen from quaternary amines, tertiary amines, amine acetates, imidazolines, amine soaps, fatty sulphates, alkyl aryl sulphonates, amine oxides, and mixtures thereof.

Hydrophobic fumed silica may be obtained by chemically modifying the surface of the silica via a chemical reaction generating a reduction in the number of silanol groups and/or a substitution of silanol groups by hydrophobic groups.

Suitable hydrophobic silicas may include, but are not limited to, silicas treated with trimethylsiloxy, dimethylsilyloxy or polydimethylsiloxane groups.

Suitable modified cellulose derivatives may be chosen from those disclosed in US2005/0100518, the entire contents of which are hereby incorporated by reference.

The elastomeric organopolysiloxanes are in general partially or completely crosslinked and optionally have a three-dimensional structure.

Suitable elastomeric organopolysiloxanes may be obtained by addition and crosslinking reaction of at least:(a) one organopolysiloxane having at least two lower alkenyl groups per molecule;(b) one organopolysiloxane having at least two hydrogen atoms linked to a silicon atom per molecule; and(c) a platinum type catalyst.

Suitable elastomeric organopolysiloxanes may also include, but are not limited to, polyorganopolysiloxanes comprising R₂SiO and RSiO_{1.5} units and optionally R₃SiO_{0.5} and/or SiO₂ units wherein the radicals R, which may be identical or different, may be chosen from a hydrogen atom, a lower alkyl group, an aryl group, and an unsaturated aliphatic group such as a vinyl.

According to a preferred embodiment of the present invention, the gelling agent is employed in an amount sufficient to carry the requisite amount of colorant necessary to enable single coat coverage of the nail polish varnish on nails. The precise amount used depends on the color, shade and style of nail polish varnish being formulated and, as a result, will be determined by those of ordinary skill in the art.

In general, the gelling agent may be present in the gelled nail polish composition in an amount of from 1 to 5% by weight, preferably from 1.5 to 4% by weight, preferably from 2 to 3.5% by weight, all weights based on the total weight of the composition.

In the event the gelling agent used is a thixotropic compound such as, for example, bentone, an activating compound may need to be employed. The gelling agent is in particular activated bentone. The activating compound is used to swell the thixotropic compound, thereby allowing more colorant to be carried by the gelled nail polish composition. In the case of clays, the activating compound is generally an acid dissolved in a polar solvent such as isopropanol. Typically, the thixotropic compound may be activated by mixing it in a solvent and, preferably, in the presence of a film-forming polymer as well. While any solvent capable of dissolving the thixotropic agent and, if desired, the film-forming polymer, may be employed, a particularly preferred solvent is isopropanol.

It should be noted, however, that in the event a thixotropic compound is used as the gelling agent, it can be employed in pre-activated form. An example thereof is stearalkonium hectorite commercially available from Elementis under the tradename Bentone 27 V.

Film-forming Polymer

The composition also contains at least one film-forming polymer. The term "film-forming polymer" is understood to mean a polymer capable of forming, alone and/or in the presence of a plasticizer, an isolable film. The film-forming polymer can be dissolved or dispersed in the form of particles in the solvent of the composition.

The film-forming polymer can be chosen from radical polymers, polycondensates and polymers of natural origin.

Suitable film-forming polymers include, but are not limited to, vinyl and acrylic polymers, polyurethanes, polyesters, alkyd resins, epoxy ester resins, cellulose polymers, such as nitrocellulose, cellulose esters, such as cellulose acetate, cellulose acetate propionate or cellulose acetate butyrate, resins resulting from the condensation of formaldehyde with an arylsulphonamide, and their mixtures.

When the composition comprises an aqueous medium, the film-forming polymer is generally present in the form of dispersed particles, thus forming a latex or pseudolatex.

Suitable film-forming polymers for use in an aqueous medium, include, but are not limited to, anionic polyurethanes, polyester-polyurethanes, polyether-polyurethanes, acrylic, acrylic/styrene, polyesters, alkyd resins, natural gums, and their mixtures.

Examples of commercially available acrylic dispersions include those sold under the tradenames Neocryl^{®} by Zeneca or Dow Latex 432^{®} by Dow Chemical. Examples of commercially available polyurethane dispersions are sold under the names "Avalure^{®}" or "Sancure^{®}" by Goodrich and "Neorez^{®}" by Avecia.

The film-forming polymer may be present in the gelled nail polish composition in an amount of from 0.5 to 20% by weight, preferably from 1 to 15% by weight, preferably from 5 to 12% by weight, based on the total weight of the composition.

Solvent

The composition according to the invention also comprises at least one solvent.

Suitable organic solvents are those which are liquid at ambient temperature and include, but are not limited to,:
- ketones such as methyl ethyl ketone, methyl isobutyl ketone, diisobutyl ketone, isophorone, cyclohexanone or acetone;
- alcohols, such as ethanol, isopropanol, diacetone alcohol, 2-butoxyethanol or cyclohexanol;
- glycols, such as ethylene glycol, propylene glycol, pentylene glycol or glycerol;
- propylene glycol ethers, such as propylene glycol monomethyl ether, propylene glycol monomethyl ether acetate or dipropylene glycol mono(n-butyl) ether;
- short-chain esters (having a total of 2 to 7 carbon atoms), such as ethyl acetate, methyl acetate, propyl acetate, n-butyl acetate or isopentyl acetate;
- alkanes, such as decane, heptane, dodecane or cyclohexane;
- aldehydes, such as benzaldehyde or acetaldehyde; and
- their mixtures.

The organic solvent is preferably chosen from short-chain esters (having a total of from 2 to 8 carbon atoms), alcohols, and their mixtures.

The at least one solvent may also comprise an aqueous medium. In that case, suitable solvent candidates include, but are not limited to, water, water -miscible solvents, such as lower monoalcohols having from 1 to 5 carbon atoms, for example ethanol, glycols having from 2 to 8 carbon atoms, C₃-C₄ ketones and C₂-C₄ aldehydes, and combinations thereof.

The at least one solvent may be present in the gelled nail polish composition in an amount of from 10 to 80% by weight, preferably from 20 to 70% by weight, preferably from 30 to 60% by weight, based on the weight of the composition.

Colorant

The composition may also comprise at least one colorant which can be chosen from pulverulent compounds and/or dyes which are soluble in the solvent of the composition.

The pulverulent compounds can be chosen from pigments and/or pearlescent agents and/or glitter generally used in nail varnishes.

The pigments can be white or colored and inorganic and/or organic. Mention may be made, among inorganic pigments, of titanium dioxide, which has optionally been surface-treated, zirconium, cerium, iron or chromium oxides, manganese violet, ultramarine blue, chromium hydrate, ferric blue or metal pigments, such as aluminum, copper or bronze. Mention may be made, among organic pigments, of carbon black, pigments of D & C type, lakes based on cochineal carmine or on barium, strontium, calcium or aluminum, or guanine.

Other pigments may also be chosen from pigments with an effect, such as particles comprising an organic or inorganic and natural or synthetic substrate, for example glass, acrylic, polyester, polyurethane or poly(ethylene terephthalate) resins, ceramics or aluminas, which substrate may or may not be covered with metal substances, such as aluminum, gold, copper or bronze, or with metal oxides, such as titanium dioxide, iron oxide or chromium oxide, or with inorganic or organic pigments, and their mixtures.

The pearlescent pigments may be chosen from white pearlescent pigments, such as mica covered with titanium oxide or with bismuth oxychloride, colored pearlescent pigments, such as titanium oxide-coated mica with iron oxides, titanium oxide-coated mica with in particular ferric blue or chromium oxide, or titanium oxide-coated mica with an organic pigment of the abovementioned type, and fluorophlogopites with iron oxides.

Other pigments may include those with goniochromatic properties, in particular liquid crystal or multilayer pigments.

The dyes are, for example, Sudan red, DC Red 17, DC Green 6, β-carotene, soybean oil, Sudan brown, DC Yellow 11, DC Violet 2, DC Orange 5 or quinoline yellow.

The colorant may also be chosen from fluorescent whitening agents.

In a preferred embodiment, the colorant is employed in an amount sufficient to facilitate one-coat coverage of the nail polish varnish onto a nail. The precise amount used depends on the color, shade and style of nail polish varnish being formulated and, as a result, will be determined by those of ordinary skill in the art.

In general, the colorant may be present in the gelled nail polish composition in an amount of up to 25% by weight, preferably from 1 to 20% by weight, preferably from 5 to 15% by weight, based on the total weight of the composition.

The gelled nail polish composition of the present invention will have a Brookfield viscosity of at least 20 poise, preferably at least 40 poise, preferably at least 60 poise.

Viscosity Reducing Agent

The viscosity reducing agent of the present invention is used to reduce/break the viscosity of the above-described gelled nail polish composition.

Once the viscosity reducing agent is combined with the gelled nail polish composition, the Brookfield viscosity, as defined above, of the resultant mixture, i.e., the nail polish varnish, is at most 30 poise, preferably at most 20 poise, preferably at most 15 poise. In one embodiment, the viscosity of the nail polish varnish is permanently reduced. However, it should be noted that so long as the viscosity of the nail polish varnish is reduced for a period of time necessary for application of the varnish onto an individual's nails, its viscosity may subsequently increase from the above-disclosed maximum viscosity values, without departing from the spirit of the invention.

According to a preferred embodiment of the present invention, the viscosity reducing agent is a chemical compound such as, for example, an alkaline compound or an acidic compound. Suitable alkaline compounds include, but are not limited to, metal hydroxides, amines, and the like.

According to one embodiment, the viscosity reducing agent, preferably an alkaline compound, is employed in an amount sufficient to rapidly reduce the viscosity of the gelled nail polish composition(s). The viscosity will typically be reduced by at least 50% of its original viscosity, i.e., its viscosity immediately prior to introduction of the viscosity reducing agent, preferably by at least 60% of its original viscosity, and more preferably by at least 70% of its original viscosity.

The viscosity reducing agent should also be capable of rapidly reducing the viscosity of the gelled nail polish composition. Thus, once it is combined with the gelled nail polish composition, the resultant nail polish varnish should achieve a desired Brookfield viscosity within 4 minutes after combination with the gelled nail polish composition, preferably within 2 minutes, and more preferably within 1 minute.

In the event the viscosity reducing agent is an alkaline compound, it will typically be employed in an amount of from 0.05% to 0.20% by weight, preferably from 0.07 to 0.10% by weight, based on the weight of the composition.

Solvent capable of solubilizing the chemical compound

In order to be more effective in rapidly breaking the viscosity, it is preferred that the chemical compound, preferably alkaline compound, be dissolved in a solvent, prior to its being combined with the above-described gelled nail polish composition. In the case of a gelled nail polish composition containing organic solvents, the chemical compound may be solubilized in a polar solvent compatible with the organic solvents. Suitable polar solvents may include, but are not limited to, ethanol, isopropanol, acetone, ethyl acetate, isobutyl acetate, butyl acetate, propyl acetate, isopropyl acetate, methyl ethyl ketone.

The solvent capable of solubilizing the chemical compound is employed in an amount sufficient to dissolve the chemical compound. Typically, this amount will range from 2 to 5% by weight, preferably from 2 to 4% by weight, preferably from 2.5 to 4% by weight, based on the weight of the composition.

It should be noted, however, that the solvent may be used in the absence of a chemical compound as the viscosity of the gelled nail polish composition may also be reduced through simple dilution. In that case, the solvent itself will serve as the viscosity reducing agent.

Auxiliaries

The composition according to the invention may additionally comprise an additional thickening agent different from the thixotropic thickening agents described above.

When the composition according to the invention comprises an aqueous medium, it may contain an additional thickening agent for an aqueous medium. This thickening agent is not capable, on its own, of giving the composition the thixotropic plastic character (nonthixotropic thickener); it makes it possible for example to adjust the viscosity of the composition in order to obtain a homogeneous flow.

When the composition comprises an aqueous medium, the additional thickening agent may be chosen from hydrophilic thickening agents (nonthixotropic thickening agent for aqueous media). Suitable hydrophilic thickening agents may include, but are not limited to, water-soluble cellulosic thickeners, guar gums, quaternized guar gums, nonionic guar gums comprising C₁-C₆ hydroxyalkyl groups, xanthan, carob, scleroglucan, gellan, rhamsan and karaya gums, alginates, maltodextrin, starch and its derivatives, hyaluronic acid and its salts, polyglyceryl (meth)acrylate polymers, polyvinylpyrrolidones, polyvinyl alcohols, crosslinked polymers and copolymers of acrylamide, and associative polymers such as associative polyurethanes.

When the composition comprises an oily medium, the additional thickening agent may be chosen from lipophilic thickening agents (nonthixotropic thickening agent for oily media).

Suitable additional lipophilic thickening agents may include, but are not limited to, alkylated guar gums, oil gelling polymers such as the polymers resulting from the polymerization or copolymerization of at least one monomer with an ethylenic group, and polyamide resins comprising alkyl groups having from 12 to 22 carbon atoms.

The additional thickening agent may be present in an amount of from 1 to 5% by weight, preferably from 1 to 4% by weight, preferably from 1 to 3% by weight, all weights based on the total weight of the composition.

The composition may additionally comprise at least one plasticizer. Suitable plasticizers include, but are not limited to, glycols and their ether or ester derivatives, esters of acids, in particular carboxylic acids, such as citrates, adipates, carbonates, tartrates, phosphates or sebacates, oxyethylenated derivatives, such as oxyethylenated oils, and their mixtures.

The plasticizer may be present in the composition according to the invention in an amount of from 1 to 12% by weight, preferably from 2 to 10% by weight, preferably from 3 to 8% by weight, all weights based on the total weight of the composition.

The composition may additionally comprise any other additive or auxiliary commonly used in cosmetic compositions and known to a person skilled in the art as being capable of being incorporated in a nail varnish composition.

Such additives or auxiliaries may be chosen from coalescents, preservatives, fragrances, oils, waxes, surfactants, antioxidants, agents for combating free radicals, spreading agents, wetting agents, dispersing agents, antifoaming agents, neutralizing agents, stabilizing agents, active principles chosen from essential oils, UV screening agents, sunscreens, moisturizing agents, vitamins, proteins, ceramides, plant extracts, fibers, and the like, and their mixtures.

Of course, a person skilled in the art will take care to choose these possible additional compounds and/or their amounts so that the properties of the composition according to the invention are not, or not substantially, detrimentally affected by the envisaged addition.

According to one embodiment of the present invention, there is provided a process for making a custom-colored cosmetic nail composition involving the steps of:(a) providing a gelled nail polish composition having a Brookfield viscosity of at least 20 poise, the composition containing: (i) at least one gelling agent; (ii) at least one film forming polymer; (iii) at least one solvent; and (iv) optionally, at least one colorant; (b) providing at least one chemical viscosity reducing agent; and (c) combining the gelled nail polish composition and the at least one chemical viscosity reducing agent to form a custom-colored nail polish varnish having a Brookfield viscosity of at most 30 poise.

The combination of the gelled nail polish composition with the at least one chemical viscosity reducing agent will, preferably, involve a certain degree of physical mixing. Thus, while a chemical compound, such as an alkaline compound, is used as the viscosity reducing agent, its combination with the gelled nail polish composition will involve some input of mechanical energy. Means for combining the gelled nail polish composition with the chemical viscosity reducing agent may be provided. For example, a spatula may be used to physically mix the gelled nail polish composition and the chemical viscosity reducing agent in order to make the nail polish varnish. It should be noted, however, that any means capable of generating mechanical energy for blending/mixing may be employed, without departing from the spirit of the invention.

The blending/mixing of the chemical viscosity reducing agent with the gelled nail polish composition is preferably performed in a re-usable/washable container, such as, for example, one made of glass. Once the nail polish varnish has been made, it may be applied onto nails in any conventional manner such as, for example, using an artist's brush.

The present invention is also directed to a nail polish varnish composition containing: (a) at least one gelling agent; (b) at least one film-forming polymer; (c) at least one solvent; (d) at least one colorant; and (e) at least one chemical viscosity reducing agent, wherein (a) and (d) are present in the composition in an amount sufficient to facilitate one-coat coverage of the nail polish varnish on nails.

According to yet another embodiment, the present invention relates to a process for making up nails involving the steps of: (a) providing a gelled nail polish composition having a Brookfield viscosity of at least 20 poise, the composition containing: (i) at least one gelling_ agent; (ii) at least one film-forming polymer; (iii) at least one solvent; (iv) at least one colorant; (b) providing at least one chemical viscosity reducing agent; (c) blending (a) and (b) to form a nail polish varnish having a Brookfield viscosity of at most 30 poise; and (d) applying a single coat of the nail polish varnish onto nails, wherein (c) is performed just prior to step (d).

Another aspect of the present invention relates to a cosmetic kit for making up nails comprising a multi-unit receptacle containing: (a) at least one unit having a gelled nail polish composition, the gelled nail polish composition containing: (i) at least one gelling agent; (ii) at least one film-forming polymer; (iii) at least one solvent; (iv) at least one colorant; (b) at least one unit containing at least one chemical viscosity reducing agent; (c) optionally, at least one container in which (a) and (b) can be blended; (d) optionally, at least tool for blending (a) and (b); and (e) optionally, at least one tool for coating nails.

According to yet another embodiment of the present invention, there is provided a multi-unit kit for making custom-colored nail polish varnishes. The kit comprises: (a) at least one unit having a first gelled nail polish composition as described above; (b) at least one unit having a second gelled nail polish composition as described above; and (c) at least one unit containing a chemical reducing agent capable of reducing the viscosity of the first and second gelled nail polish compositions.

The kit may additionally comprise a re-usable/washable container for blending/mixing the contents of the respective units, preferably a glass jar; mechanical means for blending/mixing and/or combining the ingredient contained in the respective units, preferably a small spatula; and means for applying the custom-colored nail polish varnish onto nails, preferably an artist brush for example a small artist brush. It should be understood, however, that any means suitable for effectuating the processes of the present invention may be employed without departing from the spirit of the present invention.

The units are preferably made of a flexible, non-breakable material such as, for example, plastic and may be configured in any desired shape such as, for example, a tube. It is because of this type of packaging, coupled with the highly pigmented nature of the gelled nail polish compositions, that conventional issues regarding handling and storage are, for the most part, a non-factor. The flexible containers are non-breakable and, therefore, much easier to handle and store.

It should be noted, however, that it is not necessary to use such packaging in order to effectuate the goal of being able to create custom-colored nail polishes per the process of the present invention.

The present invention will be better understood from the examples which follow, all of which are intended for illustrative purposes only, and are not meant to unduly limit the scope of the invention in any way.

Example 1: "Deep-Red" Shade Gelled Nail Polish Composition having a Brookfield viscosity of 3000 poise.

| INCLUS | Concentration (%) |
|---|---|
| TRIBUTYL CITRATE | 7.97 |
| STEARALKONIUM HECTORITE | 2.49 |
| NITROCELLULOSE (and) ISOPROPYL ALCOHOL | 12.64 |
| ACRYLATES COPOLYMER¹ | 3.02 |
| PHTHALIC ANHYDRIDE/GLYCERIN/GLYCIDYL DECANOATE COPOLYMER | 14.03 |
| ETHYL ACETATE | 7.05 |
| BUTYL ACETATE | 43.72 |
| POLOXAMER 184 | 3.04 |
| PIGMENTS | 5.95 |
| POTASSIUM HYDROXIDE | 0.08 |
| TOTAL | 100.00% |

| | |
|---|---|
| ¹ Pecorez AC 50 | |

Example 2: "Gold-Pearlescent" Shade Gelled Nail Polish Composition having a Brookfield viscosity of 4000 poise.

| INCLUS | Concentration (%) |
|---|---|
| TRIBUTYL CITRATE | 7.97 |
| STEARALKONIUM HECTORITE | 2.49 |
| NITROCELLULOSE (and) ISOPROPYL ALCOHOL | 12.64 |
| ACRYLATES COPOLYMER | 3.02 |
| PHTHALIC ANHYDRIDE/GLYCERIN/GLYCIDYL DECANOATE COPOLYMER | 14.03 |
| ETHYL ACETATE | 7.05 |
| BUTYL ACETATE | 34.54 |
| POLOXAMER 184 | 3.04 |
| PERLESCENT PIGMENTS | 15.14 |
| POTASSIUM HYDROXIDE | 0.08 |
| TOTAL | 100.00% |

A nail polish varnish in accordance with the present invention was prepared by dispensing approximately 1 ml. of the gelled nail polish compositions of Examples 1 and 2 into separate glass mixing jars, adding 3 drops of a 0.22 molar solution of potassium hydroxide to each mixing jar, and stirring the contents of each mixing jar with a spatula to form a nail polish varnish in accordance with the present invention. Each nail polish varnish had a Brookfield viscosity of 20 poise.

## Claims

1. A process for making a nail polish varnish comprising at least the steps of:
(a) providing a gelled nail polish composition having a Brookfield viscosity of at least about 20 poise, the composition containing: (i) at least one gelling agent; (ii) at least one film forming polymer; (iii) at least one solvent; and (iv) optionally, at least one colorant;
(b) providing at least one chemical viscosity reducing agent; and
(c) combining the gelled nail polish composition, and the at least one chemical viscosity reducing agent to form a nail polish varnish having a Brookfield viscosity of at most about 30 poise.

2. The process according to the preceding claim wherein (a)(i) is activated bentone.

3. The process according to claim 1 or 2 wherein the at least one chemical viscosity reducing agent is chosen from an alkaline compound and an acidic compound.

4. The process according to any one of the preceding claims further comprising providing means for combining the gelled nail polish composition with the chemical viscosity reducing agent.

5. The process according to any one of the preceding claims wherein the nail polish varnish achieves a Brookfield viscosity of at most about 30 poise within about 4 minutes after combination of the chemical viscosity reducing agent with the gelled nail polish composition.

6. The process according to any one of the preceding claims wherein (a) (iv) is employed in an amount sufficient to facilitate one-coat coverage of the nail polish varnish onto a nail.

7. The process according to any one of the preceding claims wherein the process is performed immediately prior to application of the nail polish varnish onto nails.

8. The product of the process according to any one of claims 1 to 7.

9. A process for making up nails comprising coating the nails with the nail polish varnish obtained according to any one of claims 1 to 7.

10. A multi-unit kit for making custom-colored nail polish varnishes, the kit comprising:
(a) at least one unit having a gelled nail polish composition having a Brookfield viscosity of at least about 20 poise, the composition containing: (i) at least one gelling agent; (ii) at least one film forming polymer; (iii) at least one solvent; and (iv) at least one colorant; and
(b) at least one unit containing at least one chemical viscosity reducing agent capable of reducing the viscosity of the gelled nail polish composition.

11. The kit according to the preceding claim wherein (a)(i) is activated bentone.

12. The kit according to claim 10 or 11 wherein the at least one chemical viscosity reducing agent is chosen from an alkaline compound and an acidic compound.

13. The kit according to any one of claims 10 to 12further comprising means for combining the gelled nail polish composition with the chemical viscosity reducing agent.

14. The kit according to any one of claims 10 to 13 wherein (a)(iv) is employed in an amount sufficient to facilitate one-coat coverage of the nail polish varnish onto a nail.

15. The kit according to claim 13wherein the means for combining the gelled nail polish composition with the chemical viscosity reducing agent is an artist brush.
